# EUROPEAN PATENT APPLICATION

(11) **EP 4 265 203 A1**
(43) Date of publication of application: **25.10.2023**
(21) Application number: 21905436.8
(22) Date of filing: 18.11.2021
(51) Int. Cl.: A61B 17/22

(54) **PRESSURE WAVE BALLOON CATHETER AND MEDICAL DEVICE**

(30) Priority: 16.12.2020 CN 202011484119
(71) Applicant: Sonosemi Medical Co., Ltd., Shenzhen, Guangdong 518000 (CN)
(72) Inventor: HU, Jun, Shenzhen, Guangdong 518000 (CN); LIU, Bin, Shenzhen, Guangdong 518000 (CN); LI, Bin, Shenzhen, Guangdong 518000 (CN)
(74) Representative: Potter Clarkson
(86) International application number: PCT/CN2021/131537
(87) International publication number: WO 2022/127507

(57) **Abstract**

The present application provides a pressure wave balloon catheter and a medical instrument, including a host machine, a balloon dilating catheter, and an electrode; a front end of the balloon dilating catheter is provided with a first balloon, a side wall of the first balloon is configured to be attached to a lesion site of the vascular tissue; the electrode is arranged adjacent to the side wall of the first balloon; the host machine is connected to the electrode through a conductive wire, the host machine is configured to supply a pulse voltage to the electrode to form a pressure wave at the side wall of the first balloon. The pressure wave balloon catheter and the medical instrument provided by the present application can improve the intensity of the pressure wave acting on the lesion site and effectively improve the treatment effect.

## Description

The present application claimed priority to a Chinese Patent Application, with Application No. 202011484119.2, filed with CNIPA on December 16, 2020; the content of which is incorporated herein by reference.

### TECHNICAL FIELD

The present application relates to technical field of medical devices, and more particularly to a pressure wave balloon catheter and a medical instrument.

### BACKGROUND

Cardiovascular disease has always been one of the important factors of death in the world. In the past half a century, with the development of medical knowledge and medical technology, the mortality rate of cardiovascular disease has been greatly reduced. Balloon dilatation angioplasty has played an important role in reducing the incidence and death of obstructive tubular artery disease. Traditional catheter interventional therapy usually involves Percutaneous trans luminal angioplasty (PTA) to open calcified lesions in arterial and venous vessels. When the calcified lesion is dilated in the vascular wall due to expansion of the balloon, the balloon will gradually release pressure until the calcified lesion bursts. While at the same time, the pressure accumulated in the balloon will be released instantaneously, such that the balloon is rapidly expanded to its maximum size, which may cause some damage to the blood vessel wall.

In related technologies, electro-hydraulic lithotripsy based on high-pressure underwater discharge is clinically used to destroy calcified deposits or calculus in urethra or biliary tract. Therefore, high-pressure underwater discharge technology can also be applied to destroy the calcified lesion in blood vessels. One or more pairs of discharge electrodes are placed in the angioplasty balloon to form a set of pressure wave generator, and then the electrodes are connected to the high voltage pulse power supply host machine at the other end of the balloon dilator through conductive wires. When the balloon is placed at the calcified lesion in the blood vessel, the host machine applies a high pressure pulse to make the pressure wave generator in the balloon to release pressure. The calcified lesion in the blood vessel can be destroyed selectively by the pressure wave, and damage to the blood vessel can be avoided simultaneously.

However, the diffusion range of pressure waves in the balloon is limited, usually about 3mm, and when the distance exceeds 3mm, the intensity of pressure waves is significantly weakened, and it is difficult to effectively destroy the calcified lesion in large-diameter blood vessels.

### TECHNICAL PROBLEM

The present application provides a pressure wave balloon catheter and a medical instrument, in order to solve the problem that it is difficult to effectively destroy calcified lesion in large-diameter blood vessels due to limited diffusion range of pressure wave in the balloon in related technologies.

### SUMMARY

According to a first aspect of the present application, a pressure wave balloon catheter is provided, which includes a host machine, a balloon dilating catheter, and an electrode; the balloon dilating catheter includes a catheter and a first balloon arranged at a front end of the catheter; a rear end of the catheter is adjacent to the host machine, the front end of the catheter is configured to be extended into a vascular tissue, a side wall of the first balloon is configured to be attached to a lesion site of the vascular tissue; the electrode is arranged adjacent to the side wall of the first balloon; the host machine is connected to the electrode through a conductive wire, the host machine is configured to supply a pulse voltage to the electrode to form a pressure wave at the side wall of the first balloon.

In an embodiment provided by the present application, by arranging the electrode at a position adjacent to the side wall of the first balloon, since the side wall of the first balloon is attached to the lesion site, thus when the pulse voltage is provided to the electrode through the host machine, the shock wave or pressure wave generated by the electrode is closer to the location of the lesion, and there is no need to spread a longer distance, which can improve the intensity of the pressure wave acting on the location of the lesion and effectively improve the treatment effect.

In a possible embodiment, the electrode is arranged at an inner side wall of the first balloon.

In this way, the electrode does not increase the overall diameter of the first balloon and can ensure the passage capacity of the first balloon in the vascular tissue.

In a possible embodiment, the pressure wave balloon catheter further includes a second balloon, the second balloon is arranged at an outer side wall of the first balloon, a diameter of the second balloon is less than a diameter of the first balloon, and the electrode is arranged in the second balloon.

In this way, for the large-diameter blood vessel, the first balloon can support the second balloon, such that the electrode is located close to or approach to the lesion site, and the lesion site of the large blood vessels can be effectively treated.

In a possible embodiment, the first balloon is a compliant balloon and the second balloon is a non-compliant balloon.

In a possible embodiment, a conductive liquid is reserved in the second balloon.

In this way, the conductive liquid fills a gap between the electrodes, which can effectively reduce the breakdown voltage of the electrodes and effectively protect the electrodes.

In a possible embodiment, the pressure wave balloon catheter further includes a third balloon, the third balloon is arranged in the first balloon, an outer wall of the third balloon is adjacent to an inner wall of the first balloon; and the electrode is arranged at the outer wall of the third balloon.

In this way, the electrode can be moved adjacent to the side wall of the first balloon through the expansion of the third balloon, so that the electrode can approach to the lesion site and effectively treat the lesion site.

In a possible embodiment, the first balloon is a non-compliant balloon and the third balloon is a compliant balloon.

In a possible embodiment, the electrode is arranged at an end of a sectional diameter of the first balloon.

In this way, it can effectively treat the uneven lesion sites in the vascular tissue.

In a possible embodiment, multiple sets of electrodes are provided, and the multiple sets of electrodes are uniformly arranged in a circumferential direction along a section of the first balloon.

According to a second aspect of the present application, a medical instrument is provided, and the medical instrument includes the pressure wave balloon catheter according to any one of above possible embodiments.

The structure of the present application and other purposes and beneficial effects will be explained in detail in conjunction with the drawings to ensure that the description of preferred embodiments is more clearly understood.

### BRIEF DESCRIPTION OF THE DRAWINGS

In order to explain the embodiments of the present application more clearly, a brief introduction regarding the accompanying drawings that need to be used for describing the embodiments of the present application or the prior art is given below; it is obvious that the accompanying drawings described as follows are only some embodiments of the present application, for those skilled in the art, other drawings can also be obtained according to the current drawings on the premise of paying no creative labor.
FIG. 1 is a first structural schematic view of a pressure wave balloon catheter provided by an embodiment of the present application;
FIG. 2 is a second structural schematic view of a pressure wave balloon catheter provided by an embodiment of the present application;
FIG. 3 is a third structural schematic view of a pressure wave balloon catheter provided by an embodiment of the present application;
FIG. 4 is a cross-sectional view along line A-A of FIG. 3;
FIG. 5 is a fourth structural schematic view of a pressure wave balloon catheter provided by an embodiment of the present application;
FIG. 6 is a fifth structural schematic view of a pressure wave balloon catheter provided by an embodiment of the present application;
FIG. 7 is a first state of a sixth structural schematic view of a pressure wave balloon catheter provided by an embodiment of the present application;
FIG. 8 is a second state of a sixth structural schematic view of a pressure wave balloon catheter provided by an embodiment of the present application.

In the drawings, the reference signs are listed:
1-pressure wave balloon catheter;
10-balloon dilating catheter; 20-electrode; 30-second balloon; 40-third balloon;
11-catheter; 12-first balloon.

### DETAILED DESCRIPTION

In order to make the purpose, technical features and advantages of the present application be more obvious and more understandable, technical solutions in the embodiments of the present application will be described clearly and comprehensively with reference to accompanying drawings in the embodiments, it is obvious that, the embodiments described below are merely part of the embodiments of the present application, but not the whole of the embodiments. Based on the embodiments in the present application, some other embodiments, which are obtained by one of ordinary skill in the art at the premise of paying no creative labor, are all included in the protection scope of the present application.

In the description of the embodiments of the present application, terms "the first" and "the second" are only used in describe purposes, and should not be considered as indicating or implying any relative importance, or impliedly indicating the number of indicated technical features. As such, technical feature(s) restricted by "the first" or "the second" can explicitly or impliedly comprise one or more such technical feature(s). In the description of the present application, "a plurality of" means two or more, unless there is additional explicit and specific limitation.

In the present application, unless there is additional explicit stipulation and limitation, terms such as "mount", "connect with each other", "connect", "fix", and so on should be generally interpreted, for example, "connect" can be interpreted as being fixedly connected, detachably connected, or connected integrally; "connect" can be further interpreted as being directly connected or indirectly connected through intermediary, or being internal communication between two components or an interaction relationship between the two components. For the one of ordinary skill in the art, the specific meanings of the aforementioned terms in the present application can be interpreted according to specific conditions.

In the present application, unless expressly stated and limited otherwise, the first feature being located "above" or "below" the second feature may be a direct contact between the first feature and the second feature, or an indirect contact between first feature and the second feature through an intermediate medium. Moreover, the first feature being located "above" the second feature can mean that the first feature is directly above or diagonally above the second feature, or simply means that the first feature is higher in level than the second feature. The first feature being located "below" the second feature can mean that the first feature is directly below or diagonally below the second feature, or simply means that the first feature is lower in level than the second feature.

In the description of the present application, it needs to be understood that, directions or location relationships indicated by terms such as "inside", "outside", "up", "down", "front", "rear", and so on are the directions or location relationships shown in the accompanying figures, which are only intended to describe the present application conveniently and simplify the description, but not to indicate or imply that an indicated device or component must have specific locations or be constructed and manipulated according to specific locations; therefore, these terms shouldn't be considered as any limitation to the present application.

Cardiovascular disease has always been one of the important factors of death in the world. In the past half a century, with the development of medical knowledge and medical technology, the mortality rate of cardiovascular disease has been greatly reduced. Balloon dilatation angioplasty has played an important role in reducing the incidence and death of obstructive tubular artery disease. Traditional catheter interventional therapy usually involves Percutaneous trans luminal angioplasty (PTA) to open calcified lesions in arterial and venous vessels. When the balloon dilates the calcified lesion in the vascular wall, the balloon will gradually release pressure until the calcified lesion bursts. While at the same time, the pressure accumulated in the balloon will be released instantaneously, such that the balloon is rapidly expanded to its maximum size, which may cause some damage to the blood vessel wall.

In recent years, with the development of the electrohydraulic technology, a kind of electrohydraulic lithotripsy based on high pressure underwater discharge has gradually emerged. The electrohydraulic technology is a kind of technology to treat calcified lesion by using the "electrohydraulic effect" to form a shock wave or a pressure wave in the liquid. The main principle of the "electrohydraulic effect" is that the electrons in the liquid between the electrodes are accelerated and the liquid molecules adjacent to the electrodes are ionized under the action of a high voltage and strong electric field. The electrons ionized in the liquid will be accelerated by the strong electric field between the electrodes to ionize more electrons, so as to form an electron avalanche. A plasma channel is formed in a region where the liquid molecules are ionized. As the ionization region expands, a discharge channel is formed between the electrodes, and the liquid is broken down.

After the discharge channel is generated, due to the small discharge resistance, a large discharge current will be generated, and the liquid around the discharge channel is heated by the discharge current, so that the liquid is vaporized and expanded rapidly. The rapidly expanded gas chamber extends in the liquid medium or produces a powerful shock wave. With the difference of discharge current and discharge time, the shock wave acts on the surrounding medium in the form of impulse or shock pressure.

A set of pressure wave generator is formed by placing a pair or pairs of discharge electrodes in the angioplasty balloon, and then the electrodes are connected to the high voltage pulse power supply host machine at the other end of the balloon dilating catheter. When the balloon is placed at the calcified lesion in the blood vessel, the host machine applies a high pressure pulse to make the pressure wave generator in the balloon release pressure. The pressure wave can selectively destroy the calcified lesion in the blood vessel, and at the same time, it can avoid causing damage to the blood vessel.

For example, SHOCKWAVE MEDICAL uses the shock wave or the pressure wave to remove the calcified lesions in the blood vessel. It is usually necessary to produce a high pressure pulse in the human body, the filled liquid in the balloon is promoted to produce bubbles through the generated high pressure pulse, and the bubbles act on the balloon wall when are ruptured, and then act on the calcified lesions, so as to achieve the purpose of crushing the calcified lesions.

However, the calcified lesions in the blood vessel are often more complex, for example, the clinical calcified lesion sites re often not evenly distributed on the cross section of the blood vessel, but there is a certain eccentricity. In the treatment of non-uniform calcified lesions, there may be a risk that the uncalcified area will be repeatedly impacted by the pressure wave or the shock wave. Moreover, the effective pressure range of the pressure wave balloon catheter is small, and the best effect is usually around 3mm, and the pressure wave intensity is significantly decreased when exceeds the range, which may not be able to effectively crush the calcified lesion. Therefore, it is difficult to effectively treat large-diameter vessels or eccentric lesions.

In order to solve the above-mentioned technical problems, a pressure wave balloon catheter is provided according to the first aspect of the present application. The main idea is to arrange the electrode at the side wall of the balloon adjacent to the front end of the balloon dilating catheter. In this way, when the side wall of the balloon is attached to the lesion site, the distance between the electrode and the lesion site is smaller. The shock wave or the pressure wave generated by the pulse voltage provided by the host machine has a small diffusion distance to the lesion site, which can effectively destroy the lesion site. In addition, the shock wave or the pressure wave is far away from the normal blood vessel wall and will not affect the normal blood vessel wall.

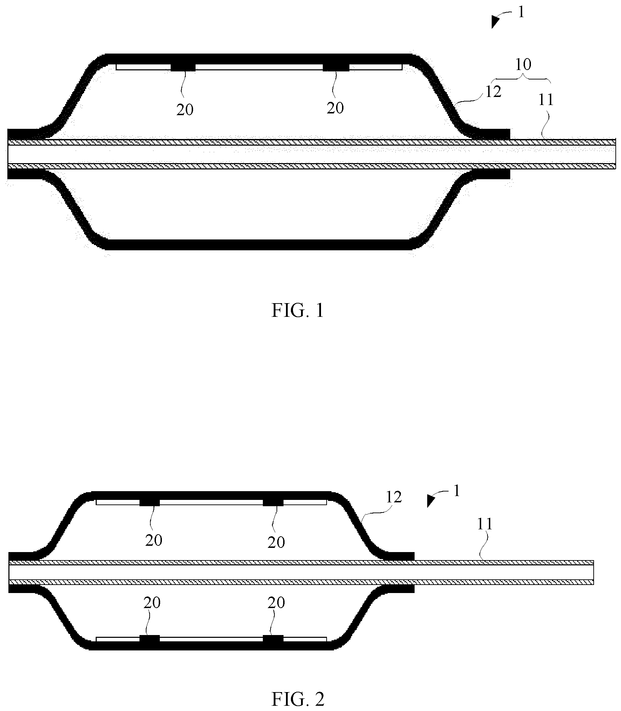

Specifically, as shown in FIG. 1, the FIG. 1 is a first structural schematic view of a pressure wave balloon catheter provided by an embodiment of the present application. A pressure wave balloon catheter 1 provided in an embodiment of the present application includes a host machine (not shown in the figure), a balloon dilating catheter 10, and an electrode 20.

Specifically, in the embodiment of the present application, the host machine can be a pulse power supply, and the pulse power supply can be a single positive pulse power supply, or a double positive or negative pulse power supply. In the embodiment, the positive pulse turn-on time width (that is, the positive pulse width) and the negative turn-on time width (that is, the negative pulse width) of the positive and negative pulse power supplies can be adjusted in the full cycle respectively.

It is understood that the pulse mode of the pulse power supply in the embodiment of the present application may be a square-wave pulse, also known as a single pulse. The single pulse power supply generally outputs unidirectional pulse current with constant parameters.

In some possible embodiments of the present application, the pulse power supply can also be a double pulse power supply or a multi-pulse power supply.

It is understood that in embodiments of the present application, the balloon dilating catheter 10 can include a catheter 11, and a first balloon 12 is arranged at the front end of the catheter 11.

In specific use, the first balloon 12 is guided to the lesion site of the blood vessel through the catheter 11, thus providing targeted treatment to the lesion site.

The side wall of the first balloon 12 is mainly used to attach to the lesion site of the vascular tissue. In this way, when the shock wave or the pressure wave is generated, the shock wave or the pressure wave will affect the side wall of the first balloon 12, thus affecting the calcified lesion and causing the calcified lesion to rupture.

Specifically, in the embodiment of the present application, the electrode 20 is arranged at a position adjacent to the side wall of the first balloon 12. The electrode 20 is electrically connected to the host machine through a conductive wire. In this way, the host machine can provide a pulse voltage to the electrode 20, and after the electric charges are accumulated on the electrode 20, the breakdown effect can be caused, thus forming the shock wave or the pressure wave in the first balloon 12.

It is understood that in embodiments of the present application, the electrode 20 can be made of materials such as a stainless steel, a copper, a silver or a tungsten that are visible under the imaging device, such as, the material is visible in X-rays. In this way, the positions of the electrode 20 and the first balloon 12 in the blood vessel can be clearly located, and the lesion site can be accurately treated.

Optionally, in the embodiments of the present application, the electrode 20 can be arranged inside the first balloon 12; the electrode 20 can further be arranged on the outside of the first balloon 12.

It is understood that the first balloon 12 can be filled with a liquid conductive medium when the electrode 20 is arranged inside the first balloon 12; the liquid conductive medium can fill the gap between the positive electrode and the negative electrode of electrode 20, and the breakdown voltage between the positive electrode and the negative electrode is reduced; which can effectively protect the electrode 20 and improve the service life of the electrode 20.

In the case that the electrode 20 is located outside the first balloon 12, an insulating layer can be wrapped around the electrode 20 or another balloon can be added. At this time, the first balloon 12 can be used as a support member in the blood vessel to support the electrode 20 adjacent to, close to, or approach to the lesion site. Thus, the shock wave or the pressure wave generated can act more effectively on the lesion site and obtain better treatment effect.

In the embodiments of the present application, the electrode 20 is arranged at a position adjacent to the side wall of the first balloon 12, since the side wall of the first balloon 12 is attached to the lesion site, thus when the pulse voltage is provided to the electrode 20 through the host machine, the shock wave or pressure wave generated by the electrode 20 is closer to the location of the lesion, and there is no need to spread a longer distance, which can improve the intensity of the pressure wave acting on the location of the lesion and effectively improve the treatment effect.

Optionally, as shown in FIG. 1, in an embodiment of the present application, the electrode 20 is arranged on the inner wall of the first balloon 12.

Specifically, in the embodiment of the present application, the electrode 20 can be arranged on the inner wall of the first balloon 12 by means of pasting. For example, the electrode 20 is attached to the inner wall of the first balloon 12 by means of a double-sided adhesive, an epoxy resin or a phenolic resin, etc.

It is understood that the interior of the first balloon 12 can be filled with a saline solution, which can be a normal saline or a mixture solution of a normal saline and a contrast agent.

In some possible examples, the electrode 20 can further be embedded in the inner wall of the first balloon 12; for example, when forming the first balloon 12, the electrode 20 is formed integrally on the inner wall of the first balloon 12.

As mentioned above, the lesion sites in the blood vessel are often not evenly distributed on the section of the blood vessel, but there is a certain eccentricity and other conditions. In order to be able to effectively treat the eccentric lesion site, continue to refer to FIG. 1, in the embodiment of the present application, the electrode 20 is arranged at one end of the sectional diameter of the first balloon 12.

It is understood that since the electrode 20 is made of materials such as a stainless steel, a copper, a silver, a gold or a tungsten that is visible in X-rays. Therefore, after the first balloon 12 is guided to the lesion site through the catheter 11, the position of the electrode 20 can be observed by a contrast device, and the position of the electrode 20 can be adjusted to the position adjacent to the lesion, that is, the side wall of the first balloon 12 adjacent to the electrode 20 is attached to the lesion. The pulse voltage is provided to the electrode 20 through the host machine, and the shock wave or the pressure wave generated by the electrode 20. It does not need to spread over a long distance, and can form an effective treatment for the lesion site.

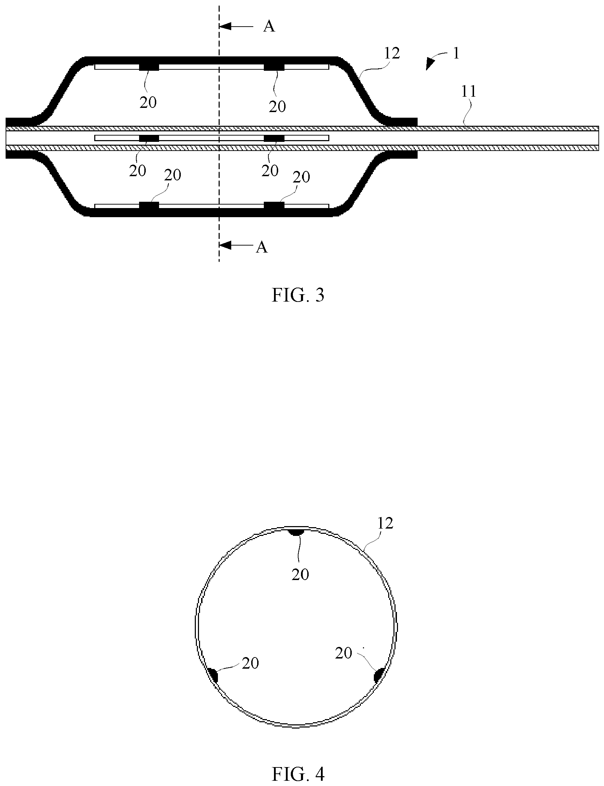

In some other possible embodiment, as shown in FIGS. 2 to 4, FIG. 2 is a second structural schematic view of a pressure wave balloon catheter provided by an embodiment of the present application; FIG. 3 is a third structural schematic view of a pressure wave balloon catheter provided by an embodiment of the present application; and FIG. 4 is a cross-sectional view along line A-A of FIG. 3. In the embodiments of the present application,multiple sets of electrodes 20 can be arranged uniformly along the circumference of the section of the first balloon 12. For example, as shown in FIG. 2, when there are two sets of electrodes 20, each of the two sets of electrodes 20 can be located at one end of the sectional diameter of the first balloon 12.

In the embodiment, in the case of the multiple sets of electrodes 20, the electrodes 20 can be uniformly arranged along the circumference of the section of the first balloon 12, as shown in FIG. 4, the three sets of electrodes 20 are arranged at an angle of 120° from each other.

It should be noted that in the case of multiple sets of electrodes 20, each set of electrodes 20 in the embodiment of the present application can be separately controlled by the host machine to provide the pulse voltage. For example, a control chip can be set up in the host machine, through which the host machine can be controlled to provide the pulse voltage to each set of electrodes 20 separately. The control chip can be a Central Processing Unit/Processor (CPU), Microcontroller Unit (MCU) or a Neural network Processing Unit (NPU), etc.; which is not defined in the embodiments of the present application.

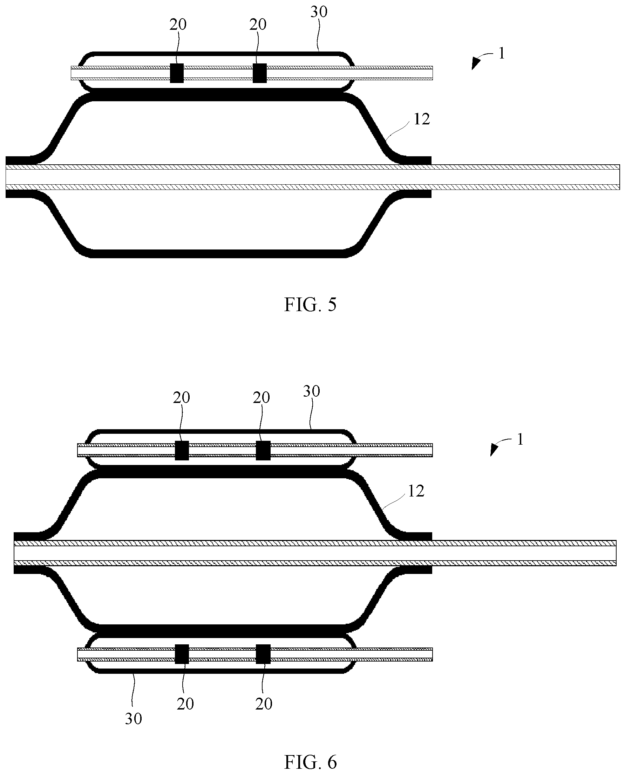

Further, as shown in FIGS. 5 and 6, in another possible embodiment, the pressure wave balloon catheter 1 further includes a second balloon 30, the second balloon 30 is arranged on the outer wall of the first balloon 12, and the diameter of the second balloon 30 is smaller than that of the first balloon 12; the electrode 20 is arranged in the second balloon 30.

Specifically, in the embodiment of the present application, the second balloon 30 is filled with a liquid conductive medium. The liquid conductive medium may be a saline solution mentioned above.

It is understood that in the embodiment of the present application, the first balloon 12 is a compliant balloon and the second balloon 30 is a non-compliant balloon.

Those skilled in the art can understand that the compliance of the balloon refers to the corresponding changes in the shape and volume of the balloon with each additional atmospheric pressure when the balloon is filled, and is an indicator of the stretching ability of the balloon. The non-compliant balloon is mainly used to treat the calcified lesion.

After the catheter 11 guides the first balloon 12 and the second balloon 30 to the lesion site, the first balloon 12 can be filled with the normal saline to fill the lumen of the blood vessel, so as to support and fix the second balloon 30 and to fix the second balloon 30 adjacent to the lesion site. The first balloon 12 is arranged to be a compliant balloon, and the diameter of the first balloon 12 varied greatly with the expansion pressure. Therefore, the first balloon 12 can be adapted to different diameters of blood vessels, which expands the scope of application of pressure wave balloon catheter1, and doesn't affect the walls of the blood vessels. Moreover, since the first balloon 12 supports the second balloon 20 adjacent to the lesion site, that is, the electrode 20 is arranged adjacent to the lesion site but far from the center of the blood vessel. In this way, while ensuring that the lesion site is within the effective range of the shock wave or pressure wave, it can also make the normal blood vessels far away from the shock wave or pressure wave, and the shock wave or pressure wave has less impact on the normal blood vessel tissue, so as to avoid the damage to the normal blood vessel tissue.

In the embodiment, a plurality of the second balloons 30 can be provided, and the plurality of the second balloons 30 are uniformly arranged in the circumferential direction of the first balloon 12.

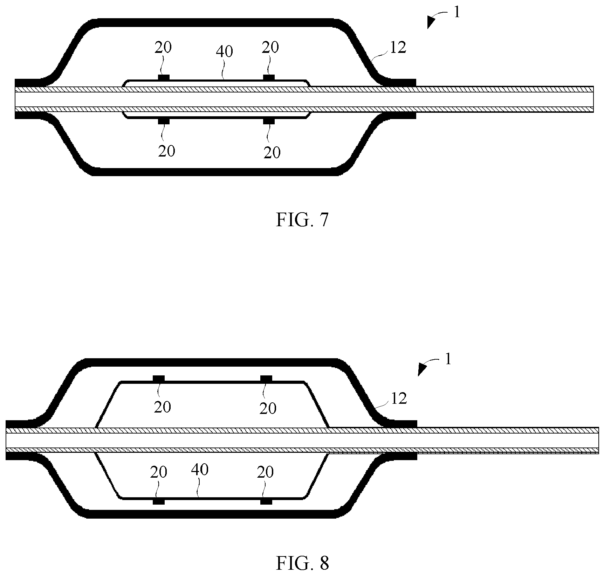

Further, as shown in FIG. 7 and FIG. 8, FIG. 7 is a first state of a sixth structural schematic view of a pressure wave balloon catheter provided by an embodiment of the present application; and FIG. 8 is a second state of a sixth structural schematic view of a pressure wave balloon catheter provided by an embodiment of the present application. The pressure wave balloon catheter 1 further includes a third balloon 40, the third balloon 40 is arranged in the first balloon 12. When the third balloon 40 expands, the outer wall of the third balloon 40 is arranged adjacent to the inner wall of the first balloon 12; the electrode 20 is arranged on the outer wall of the third balloon 40.

Specifically, the first balloon 12 is a non-compliant balloon, and the third balloon 40 is a compliant balloon.

Further, in the embodiment of the present application, the first balloon 12 can be filled with a saline solution, and the third balloon 40 can be expanded by filling with a normal saline.

For specific use, as shown in FIG. 8, the first balloon 12 is firstly guided to the lesion site through the catheter 11, then the third balloon 40 is filled with the normal saline to expand the third balloon 40, and the electrode 20 on the outer wall of the third balloon 40 is driven to the inner wall of the first balloon 12.

In this way, the electrode 20 can be arranged adjacent to the location of the lesion, which can effectively treat the lesion.

According to the second aspect of the present application, a medical instrument is provided, and the medical instrument includes the pressure wave balloon catheter 1 provided in any of the optional embodiments of the first aspect of the present application.

The above is only the specific implementation of the present application, but the protection scope of the present application is not limited to this, those skilled in the art can easily think of changes or replacements within the technical scope disclosed in the present application, should be covered by the protection scope of the present application. Therefore, the protection scope in the present application shall be governed by the protection scope of the claims.

## Claims

1. A pressure wave balloon catheter, comprising:
a host machine;
a balloon dilating catheter (10), comprising a catheter (11) and a first balloon (12) arranged at a front end of the catheter (11); wherein a rear end of the catheter (11) is adjacent to the host machine, the front end of the catheter (11) is configured to be extended into a vascular tissue, a side wall of the first balloon (12) is configured to be attached to a lesion site of the vascular tissue; and
an electrode (20), arranged adjacent to the side wall of the first balloon (12);
wherein the host machine is connected to the electrode (20) through a conductive wire, the host machine is configured to supply a pulse voltage to the electrode (20) to form a pressure wave at the side wall of the first balloon (12).

2. The pressure wave balloon catheter according to claim 1, wherein the electrode (20) is arranged at an inner side wall of the first balloon (12).

3. The pressure wave balloon catheter according to claim 1, wherein the pressure wave balloon catheter further comprises a second balloon (30), the second balloon (30) is arranged at an outer side wall of the first balloon (12), and a diameter of the second balloon (30) is less than a diameter of the first balloon (12), and the electrode (20) is arranged in the second balloon (30).

4. The pressure wave balloon catheter according to claim 3, wherein the first balloon (12) is a compliant balloon and the second balloon (30) is a non-compliant balloon.

5. The pressure wave balloon catheter according to claim 3, wherein a conductive liquid is reserved in the second balloon (30).

6. The pressure wave balloon catheter according to claim 1, wherein the pressure wave balloon catheter further comprises a third balloon (40), the third balloon (40) is arranged in the first balloon (12), an outer wall of the third balloon (40) is adjacent to an inner wall of the first balloon (12); and the electrode (20) is arranged at the outer wall of the third balloon (40).

7. The pressure wave balloon catheter according to claim 6, wherein the first balloon (12) is a non-compliant balloon and the third balloon (40) is a compliant balloon.

8. The pressure wave balloon catheter according to any one of claims 1 to 7, wherein the electrode (20) is arranged at an end of a sectional diameter of the first balloon (12).

9. The pressure wave balloon catheter according to any one of claims 1 to 7, wherein multiple sets of electrodes (20) are provided, and the multiple sets of electrodes (20) are uniformly arranged in a circumferential direction along a section of the first balloon (12).

10. A medical instrument, comprising the pressure wave balloon catheter (1) according to any one of claims 1 to 9.
